# EUROPEAN PATENT APPLICATION

(11) **EP 4 659 711 A1**
(43) Date of publication of application: **10.12.2025**
(21) Application number: 24749509.6
(22) Date of filing: 10.01.2024
(51) Int. Cl.: A61F 2/38, A61F 2/46

(54) **ARTIFICIAL KNEE JOINT PAD AND METHOD FOR CONSTRUCTING POSTERIOR CRUCIATE BED OF ARTIFICIAL KNEE JOINT PAD**

(30) Priority: 02.02.2023 CN 202310120363
(71) Applicant: Beijing Naton Medical Technology Holdings Co., Ltd., Beijing 100094 (CN); Tianjin Zhengtian Medical Instrument Co., Ltd., Airport Economic Zone Tianjin, 300308 (CN)
(72) Inventor: SONG, Dayong, Beijing 100094 (CN); WANG, Jian, Beijing 100094 (CN); XU, Shufu, Beijing 100094 (CN); DING, Yubao, Beijing 100094 (CN)
(74) Representative: advotec.
(86) International application number: PCT/CN2024/071526
(87) International publication number: WO 2024/160027

(57) **Abstract**

An artificial knee joint pad and a method for constructing a posterior cruciate bed of an artificial knee joint pad. The artificial knee joint pad comprises a body, wherein the body is fixed to an upper resection surface of a tibia and comprises a medial support portion and a lateral support portion, which are arranged opposite to each other in a width direction of the body, the medial support portion and the lateral support portion respectively bearing a medial condyle and a lateral condyle of a femur; and a posterior side surface of the body is a posterior condyle surface, a recess opening backwards and used for avoiding a posterior cruciate ligament of a human body is formed in the posterior condyle surface, the recess is positioned between the medial support portion and the lateral support portion, and a surface of the recess is recessed inwards to form a posterior cruciate bed, and a surface of the posterior cruciate bed extends obliquely upwards in a direction away from the posterior condyle surface.

## Description

### CROSS-REFERENCE TO RELATED APPLICATION

The present application is based on and claims the benefit of priority to Chinese patent application No. 202310120363.8, filed on February 02, 2023, the content of which is incorporated herein by reference in its entirety for all purposes.

### FIELD

The present invention relates to the technical field of medical prostheses, and in some embodiments, relates to a bearing for an artificial knee joint and a method for constructing a posterior cruciate bed of the artificial knee joint bearing.

### BACKGROUND

At present, a total knee arthroplasty is mainly divided into two types, which are posterior cruciate ligament retaining type and posterior cruciate ligament replacement type. Compared with posterior cruciate ligament replacement type, the posterior cruciate ligament retaining type preserves more native bone and, and by retaining the posterior cruciate ligament, provides greater stability during patient's activity.

In the related art, a bearing of prostheses of the posterior cruciate ligament retaining type only avoid a stopping point of the posterior cruciate ligament without providing a comprehensive avoidance for the entire posterior cruciate ligament, which results in cutting the ligament at a edge of the ligament avoidance area of the bearing and is easy to damage the posterior cruciate ligament or cause patient's discomfort during motion.

### SUMMARY

Embodiments of the present invention provide a bearing for an artificial knee joint, including a body having an inner support portion configured to bear a medial condyle of a femur and an outer support portion configured to bear a lateral condyle of the femur; a posterior condyle surface of the body has a recess opening posteriorly, and the recess is located between the inner support portion and the outer support portion and is configured to avoid a posterior cruciate ligament of a human body, a surface of the recess is recessed inward to form a posterior cruciate bed, and a surface of the posterior cruciate bed extends obliquely upward in a direction facing away from the posterior condyle surface. As a result, the surface of the recess is recessed inward to form the posterior cruciate on the basis of the recess, and the obliquely arranged posterior cruciate bed is consistent with the posterior cruciate ligament, so that a space configured to accommodate the posterior cruciate ligament may be formed above the posterior cruciate bed, so that the movement of the posterior cruciate ligament may not interfere with the recess, and the posterior cruciate ligament can be avoided from being cut after knee joint replacement, so that the posterior cruciate ligament can be prevented from being damaged, and patients will not feel uncomfortable after operation.

The present invention also provides a method for constructing a posterior cruciate bed of a bearing for an artificial knee joint.

The bearing for an artificial knee joint according to embodiments of the present invention includes a body configured to be fixed on an upper resection surface of a tibia and having an inner support portion and an outer support portion; wherein the inner support portion and the outer support portion are oppositely arranged in a width direction of the body and are configured to bear a medial condyle of a femur and a lateral condyle of the femur respectively; a posterior side of the body is a posterior condyle surface having a recess opening posteriorly, and the recess is located between the inner support portion and the outer support portion and is configured to avoid a posterior cruciate ligament of a human body, a surface of the recess is recessed inward to form a posterior cruciate bed, and a surface of the posterior cruciate bed extends obliquely upward in a direction facing away from the posterior condyle surface so that a space above the posterior cruciate bed is configured to match movement space of the posterior cruciate ligament of the human body.

The bearing for an artificial knee joint according to embodiments of the present invention includes a body having an inner support portion configured to bear a medial condyle of a femur and an outer support portion configured to bear a lateral condyle of the femur; a posterior condyle surface of the body has a recess opening posteriorly, and the recess is located between the inner support portion and the outer support portion and is configured to avoid a posterior cruciate ligament of a human body, a surface of the recess is recessed inward to form a posterior cruciate bed, and a surface of the posterior cruciate bed extends obliquely upward in a direction facing away from the posterior condyle surface. As a result, the surface of the recess is recessed inward to form the posterior cruciate on the basis of the recess, and the obliquely arranged posterior cruciate bed is consistent with the posterior cruciate ligament, so that a space configured to accommodate the posterior cruciate ligament may be formed above the posterior cruciate bed, so that the movement of the posterior cruciate ligament may not interfere with the recess, and the posterior cruciate ligament can be avoided from being cut after knee joint replacement, so that the posterior cruciate ligament can be prevented from being damaged, and patients will not feel uncomfortable after operation.

In some embodiments, in the width direction of the body, a center of the recess is offset towards the inner support portion or the outer support portion.

In some embodiments, the posterior cruciate bed includes an inner posterior cruciate bed and an outer posterior cruciate bed symmetrically arranged about a center of the body in the width direction of the body, the inner posterior cruciate bed is adjacent to the inner support portion, the outer posterior cruciate bed is adjacent to the outer support portion, and spaces above the inner posterior cruciate bed and the outer posterior cruciate bed are configured to respectively match movement spaces of posterior cruciate ligaments of left and right legs of the human body.

In some embodiments, the surface of the posterior cruciate bed is curved.

In some embodiments, the posterior cruciate bed is a sector-shaped structure, and in the width direction of the body, a center of the posterior cruciate bed is located at a middle position of the body.

In some embodiments, an included angle of a sector-shaped projection of the posterior cruciate bed on a horizontal plane is denoted as A, wherein 10 degrees≦ A ≦ 40 degrees; and/oran included angle between a projection of the posterior cruciate bed on a vertical plane and a horizontal plane is denoted as B, wherein 20 degrees≦ B≦ 60 degrees.

The method for constructing a posterior cruciate bed of a bearing for an artificial knee joint is provided, the posterior cruciate bed of the bearing for the artificial knee joint being the posterior cruciate bed of the bearing for the artificial knee joint according to claim 1, and the method including: defining a first projection plane parallel to a bottom surface of the body, and defining a point, which is adjacent to the outer support portion, of projection points of a junction of the posterior condyle surface and the recess on the first projection plane, as a starting point of a generatrix; constructing the generatrix which extends obliquely upwards and points to the inner support portion by taking the starting point of the generatrix as an origin; and generating an entity with a polygonal section according to the constructed generatrix, and cutting the bearing at a junction area between the entity and the bearing by the entity to form the posterior cruciate bed.

According to the method for constructing a posterior cruciate bed of a bearing for an artificial knee joint, the structure of the bearding of the present application are formed by constructing the generatrix by the selected starting point of the generatrix, generating an entity according to the generatrix, and cutting the body to form the posterior cruciate bed with an accurate size.

In some embodiments, the first projection plane is located 2mm to 15mm below a lowest point of an upper surface of the outer support portion; and/or the polygonal section is round or square.

In some embodiments, constructing the generatrix which extends obliquely upwards and points to the inner support portion by taking the starting point of the generatrix as the origin includes: defining a plane orthogonal to the first projection plane as a second projection plane, and a center line of the body in the width direction of the body is located at the second projection plane; and constructing a first projection line forming an included angle a with the second projection plane on the first projection plane, constructing a second projection line forming an included angle b with the first projection plane on the second projection plane, and deducing a spatial position of the generatrix by taking the first projection line and the second projection line as projections of the generatrix.

In some embodiments, 5 degrees≦ a ≦ 20 degrees, and 20 degrees≦ 60 degrees.

### BRIEF DESCRIPTION OF THE DRAWINGS

FIG. 1 is a schematic structural diagram of a bearing for an artificial knee joint according to an embodiment of the present application.
FIG. 2 is an isometric view of a bearing for an artificial knee joint according to an embodiment of the present application.
FIG. 3 is a front view of a bearing for an artificial knee joint according to an embodiment of the present application.
FIG. 4 is an isometric view of a bearing for an artificial knee joint according to an embodiment of the present application, showing a generatrix.
FIG. 5 is a schematic structural diagram of a bearing for an artificial knee joint according to an embodiment of the present application.
FIG. 6 is a schematic structural diagram of a bearing for an artificial knee joint according to an embodiment of the present application, showing a projection of a generatrix on a horizontal projection plane.
FIG. 7 is a schematic structural diagram of a bearing for an artificial knee joint according to an embodiment of the present application, showing a projection of a generatrix on a vertical projection plane.
FIG. 8 is a schematic structural view of a bearing for an artificial knee joint according to another embodiment of the present application.
FIG. 9 is a schematic structural view of a bearing for an artificial knee joint according to another embodiment of the present application.
FIG. 10 is a cross-sectional view of a bearing for an artificial knee joint in FIG. 9.

Reference numerals:
body- 1, inner support portion- 2, outer support portion- 3, recess- 4, posterior cruciate bed- 5, inner posterior cruciate bed- 51, outer posterior cruciate bed- 52, first projection surface- 6, generatrix- 7, starting point of a generatrix- 8, posterior condyle surface- 9.

### DETAILED DESCRIPTION

Hereinafter, embodiments of the present application will be described in detail, examples of which are illustrated in the accompanying drawings. Embodiments described below by referring to the attached drawings are illustrative and are intended to explain the application, but not to be construed as limitations of the application.

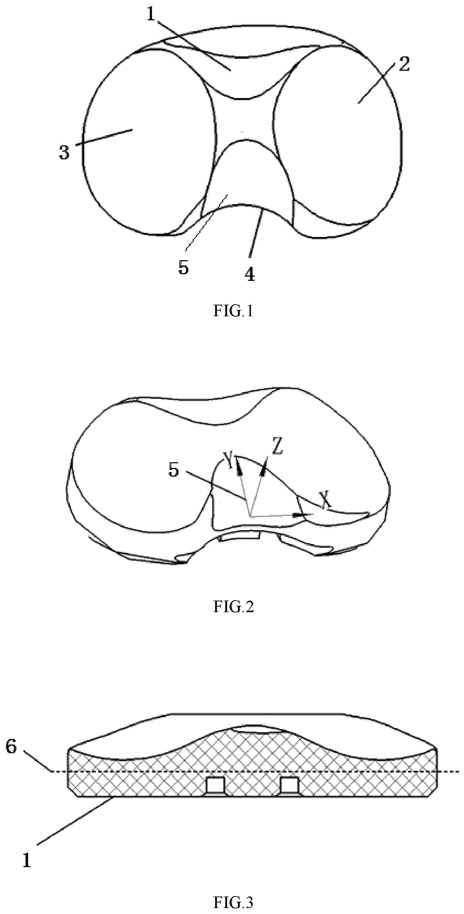

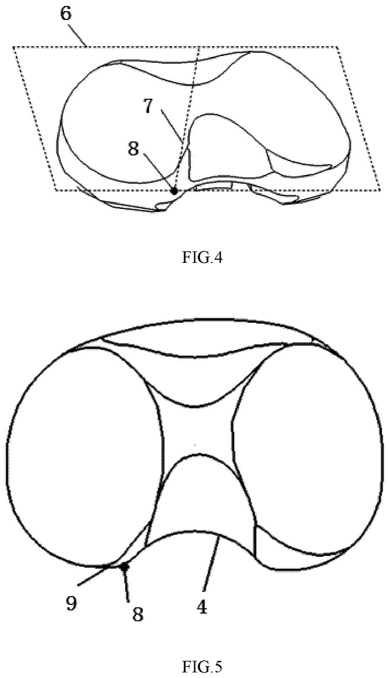

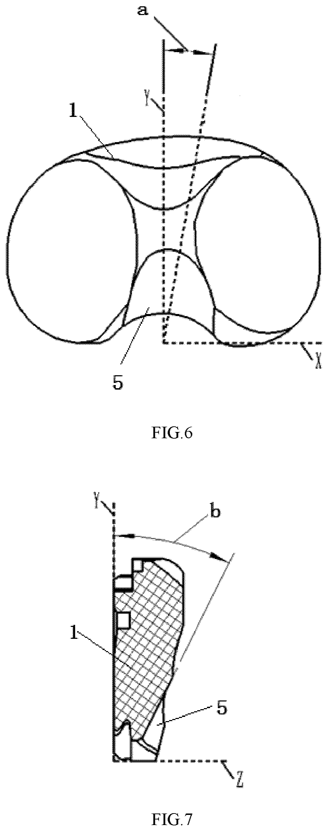

As shown in FIG. 1- FIG. 10, a bearing for an artificial knee joint in the present application includes a body 1 fixed on an upper resection surface of tibia and having an inner support portion 2 and an outer support portion 3, which support a medial condyle and a lateral condyle of femur respectively. A posterior side of the body 1 is a posterior condyle surface 9 formed with a recess 4 that opens posteriorly, and the recess 4 is located between the inner support portion 2 and the outer support portion 3. The recess 4 is used to avoid a posterior cruciate ligament of human body, and a surface of the recess 4 is depressed inward to form a posterior cruciate bed 5, and a surface of the posterior cruciate bed 5 extends obliquely upward in a direction facing away from the posterior condyle surface 9 so that a space above the posterior cruciate bed 5 matches movement space of the posterior cruciate ligament of human body.

It should be noted that the posterior cruciate ligament will move upward and downward in space along with the different motion postures of the human body, the traditional bearing avoids the posterior cruciate ligament only through the recess on the posterior condyle surface 9, but when the posterior cruciate ligament swings up and down, the edge of the recess will contact the posterior cruciate ligament to cut the posterior cruciate ligament, which will easily cause discomfort to the human body or directly damage the posterior cruciate ligament. In the present application, a posterior cruciate bed 5 is further constructed on the basis of the recess 4. If the direction of the bed surface of the posterior cruciate bed 5 obliquely arranged keeps consistent with the posterior cruciate ligament, a space configured to accommodate the posterior cruciate ligament can be formed above the posterior cruciate bed 5, and the up and down swing of the posterior cruciate ligament will not form cut with the edge of the posterior cruciate bed 5 since the space matches the movement space of the posterior cruciate ligament.

In the bearing for the artificial knee joint of embodiments of the present application, the body 1 has an inner support portion 2 for supporting the medial condyle and an outer support portion 3 for supporting the lateral condyle, and the posterior condyle surface 9 of the body 1 has a recess 4 which opens posteriorly, and the recess 4 is located between the inner support portion 2 and the outer support portion 3 for avoiding the posterior cruciate ligament, and the surface of the recess 4 is depressed inward to form a posterior cruciate bed 5, and the surface of the posterior cruciate bed 5 extends obliquely upward in a direction facing away from the posterior condyle surface 9, therefore, by forming the posterior cruciate bed 5 recessedly inward on the recess 4, and the bed surface of the posterior cruciate bed 5 obliquely arranged is consistent with the posterior cruciate ligament, a space capable of accommodating the posterior cruciate ligament can be formed above the posterior cruciate bed 5, so that the activity of the posterior cruciate ligament can not interfere with the recess 4 to avoid cutting the posterior cruciate ligament after knee joint replacement, so that the posterior cruciate ligament can be prevented from being damaged, and the patient will not have any uncomfortable reaction after operation.

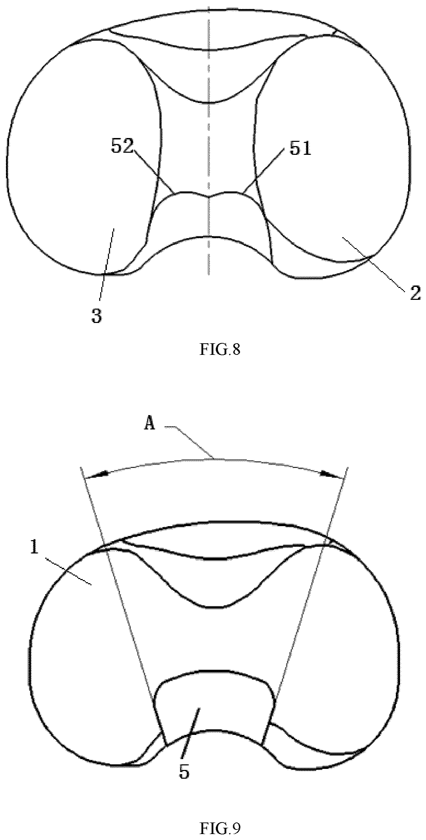

In some embodiments, as shown in FIG. 1, in a width direction of the body 1, a center of the recess 4 is offset toward the inner support portion 2 or the outer support portion 3. In some embodiments, as shown in FIG. 9, in the width direction of the body 1, the center of the recess 4 is located at the middle position between the inner support portion 2 and the outer support portion 3. In some embodiments, the setting form of the recess 4 can be adaptively selected according to the needs of different patients.

In some embodiments, as shown in FIG. 8, the posterior cruciate bed 5 includes an inner posterior cruciate bed 51 and an outer posterior cruciate bed 52 which are symmetrically arranged about the center of the body 1 in the width direction of the body 1, the inner posterior cruciate bed 51 is adjacent to the inner support portion 2, and the outer posterior cruciate bed 52 is adjacent to the outer support portion 3, and spaces above the inner posterior cruciate bed 51 and the outer posterior cruciate bed 52 respectively match the movement spaces of the posterior cruciate ligaments of the left and right legs of the human body. Therefore, the bearing of the present application can be used in knee joint replacement of left and right legs of human body without classified manufacturing, which reduces the design and production cost, and the bearing does not need to be classified and screened for the left leg or the right leg during operation, so the operation efficiency is high.

In some embodiments, the surface of the posterior cruciate bed 5 is curved. The curved surface structure has a high degree of fitting with the peripheral surface of ligament, which can play a better supporting effect on the ligament.

In some embodiments, in order to realize that the bearing of the present application can be applied to knee joint replacements of both left and right legs indiscriminately, the bearing is not limited to the two of mirror image arrangement as shown in FIG. 8. For example, as shown in FIG. 9, the posterior cruciate bed 5 has a sector-shaped structure, and the center of the posterior cruciate bed 5 is located in the middle of a total width of the bearing in the width direction of the body 1. It can be understood that the positions of the medial support portion 2 and the lateral support portion 3 are the same relative to the sector-shaped posterior cruciate bed 5, so that the posterior cruciate bed 5 can accommodate the posterior cruciate ligament when it is applied to knee joint replacement on different sides, and there is no need to carry out classified design for the left and right legs.

Further, as shown in FIG. 9, if an included angle of the sector-shaped projection of the posterior cruciate bed 5 on a horizontal plane is denoted as A, then 10 degrees≦ A ≦ 40 degrees. In other words, the opening angle of the sector-shaped posterior cruciate bed 5 is between 10 degrees and 40 degrees. Through research, the inventor found that when the opening angle of the sector-shaped posterior cruciate bed 5 is in the above range, the width of the posterior cruciate bed 5 can match the horizontal range of motion of the posterior cruciate ligament of most patients.

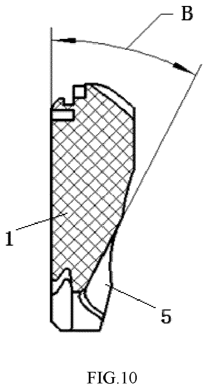

Further, as shown in FIG. 10, if an included angle between the projection of the posterior cruciate bed 5 on the vertical plane and the horizontal plane is denoted as B, then 20 degrees ≦ B ≦ 60 degrees. In other words, the tilt angle of the posterior cruciate bed 5 is between 20 degrees and 60 degrees. The inventors have found that when the tilt angle of the sector-shaped posterior cruciate bed 5 is within the above range, the extending direction of the bed surface of the posterior cruciate bed 5 is basically the same as the extending direction of the posterior cruciate ligament, which is convenient to adapt to the moving range of the posterior cruciate ligament in the vertical space.

A method for constructing the posterior cruciate bed 5 of the bearing for the artificial knee joint according to an embodiment of the present application includes: defining a first projection plane 6 (i.e., XY plane shown in FIG. 2) parallel to the bottom surface of the body 1, then defining a projection point of a junction of the posterior condyle surface 9 and the recess 4 on the projection plane 6, which is adjacent to the outer support portion 3, as a starting point 8 of a generatrix, then constructing the generatrix 7 extending obliquely upward and pointing to the inner support portion 2 by taking the starting point 8 of the generatrix as an origin, and finally based on the constructed generatrix 7, generating an entity with a polygonal section according to the constructed generatrix 7, and cutting the bearing at a junction area between the entity and the bearing by the entity to form the posterior cruciate bed 5.

According to the method for constructing the posterior cruciate bed 5 of the bearing for the artificial knee joint, aiming at the structure of the bearing in the present application, the posterior cruciate bed 5 with accurate size can be cut and formed on the body 1 by selecting the starting point 8 and constructing the generatrix 7based on the starting point and constructing a cutting entity according to the generatrix 7.

In some embodiments, the polygonal cross-section is circular or square.

Further, the first projection surface 6 is located 2 mm to 15 mm below a lowest point of the upper surface of the outer support portion 3.

Further, constructing the generatrix 7 extending obliquely upward and pointing to the inner support portion 2 by taking the starting point 8 of the generatrix as the origin includes: defining a plane orthogonal to the first projection plane 6 as the second projection plane (i.e., YZ plane shown in FIG. 2) , wherein a center line of the body 1 in the width direction of the body 1 is located on the second projection plane; constructing a first projection line forming an included angle a with the second projection plane on the first projection plane 6, and constructing a second projection line forming an included angle b with the first projection plane 6 on the second projection plane; and deducing a spatial position of the generatrix 7 by taking the first projection line and the second projection line as projections of the generatrix 7.

In some embodiments, 5 degrees≦ a ≦ 20 degrees and 20 degrees≦ b ≦ 60 degrees. That is, the constructed generatrix 7 inclines by 5 degrees to 20 degrees upward from the starting point 8 of the generatrix and inclines by 20 degrees to 60 degrees toward the inner support portion 2. Through research, the inventor found that the posterior cruciate bed 5 cut by the generatrix 7 that meets the above range can just meet the extension direction and range of motion of the posterior cruciate ligament, so as to better avoid the posterior cruciate ligament and meet the needs of knee joint replacement of most patients.

In the description of the present disclosure, it should be understood that the orientation or positional relationship indicated by the terms "center", "longitudinal", "transverse", "length", "width", "thickness", "up", "down", "front", "rear", "left", "right", "vertical", "horizontal", "top", "bottom", "inside", "outside", "clockwise", "counterclockwise", "axial", "radial" and "circumferential" and the like, is based on the orientation or positional relationship shown in the attached drawings, which is only for the convenience of describing the present disclosure and simplifying the description, and does not indicate or imply that the referred device or element must have a specific orientation and be constructed and operated in a specific orientation, so it cannot be understood as a limitation of the present disclosure.

In addition, the terms "first" and "second" are only used for purpose of description, and cannot be understood as indicating or implying relative importance or implicitly indicating the number of indicated technical features. Therefore, the feature defined as "first" or "second" may explicitly or implicitly include at least one such feature. In the description of the present disclosure, "a plurality of" means at least two, such as two, three, etc., unless otherwise specifically defined.

In the present disclosure, unless otherwise expressly defined, terms such as "install", "interconnect", "connect", "fix" shall be understood broadly, and may be, for example, fixed connections, detachable connections, or integral connections; may also be mechanical or electrical connections or intercommunication; may also be direct connections or indirect connections via intervening media; may also be inner communications or interactions of two elements. For those skilled in the art, the specific meaning of the above terms in the present disclosure can be understood according to the specific situations.

In the present disclosure, unless otherwise expressly defined and specified, a structure in which a first feature is "on" or "below" a second feature may include an embodiment in which the first feature is in direct contact with the second feature, or may further include an embodiment in which the first feature and the second feature are in indirect contact through intermediate media. Furthermore, a first feature "on", "above", or "on top of' a second feature may include an embodiment in which the first feature is right or obliquely "on", "above", or "on top of' the second feature, or just means that the first feature is at a height higher than that of the second feature, while a first feature "below", "under", or "on bottom of' a second feature may include an embodiment in which the first feature is right or obliquely "below", "under", or "on bottom of' the second feature, or just means that the first feature is at a height lower than that of the second feature.

In the description of the present disclosure, terms such as "an embodiment", "some embodiments", "an example", "a specific example" or "some examples" means that a particular feature, structure, material, or characteristic described in connection with the embodiment or example is included in at least one embodiment or example of the present disclosure. Thus, the appearances of these terms in various places throughout this specification are not necessarily referring to the same embodiment or example of the present disclosure. Furthermore, the particular features, structures, materials, or characteristics may be combined in any suitable manner in one or more embodiments or examples. In addition, without contradiction, those skilled in the art may combine and unite different embodiments or examples or features of the different embodiments or examples described in this specification.

Although the embodiments of the present disclosure have been shown and described above, it can be understood that the above embodiments are illustrative and shall not be understood as limitation to the present disclosure, and changes, modifications, alternatives and variations can be made in the above embodiments within the scope of the present disclosure by those skilled in the art.

## Claims

1. A bearing for an artificial knee joint, comprising a body configured to be fixed on an upper resection surface of a tibia and having an inner support portion and an outer support portion; wherein the inner support portion and the outer support portion are oppositely arranged in a width direction of the body and are configured to bear a medial condyle of a femur and a lateral condyle of the femur respectively; a posterior side of the body is a posterior condyle surface having a recess opening posteriorly, and the recess is located between the inner support portion and the outer support portion and is configured to avoid a posterior cruciate ligament of a human body, a surface of the recess is recessed inward to form a posterior cruciate bed, and a surface of the posterior cruciate bed extends obliquely upward in a direction facing away from the posterior condyle surface so that a space above the posterior cruciate bed is configured to match movement space of the posterior cruciate ligament of the human body.

2. The bearing for the artificial knee joint according to claim 1, wherein, in the width direction of the body, a center of the recess is offset towards the inner support portion or the outer support portion.

3. The bearing for the artificial knee joint according to claim 1, wherein the posterior cruciate bed comprises an inner posterior cruciate bed and an outer posterior cruciate bed symmetrically arranged about a center of the body in the width direction of the body, the inner posterior cruciate bed is adjacent to the inner support portion, the outer posterior cruciate bed is adjacent to the outer support portion, and spaces above the inner posterior cruciate bed and the outer posterior cruciate bed are configured to respectively match movement spaces of posterior cruciate ligaments of left and right legs of the human body.

4. The bearing for the artificial knee joint according to claim 1, wherein the surface of the posterior cruciate bed is curved.

5. The bearing for the artificial knee joint according to claim 1, wherein the posterior cruciate bed is a sector-shaped structure, and in the width direction of the body, a center of the posterior cruciate bed is located at a middle position of the body.

6. The bearing for the artificial knee joint according to claim 5, wherein an included angle of a sector-shaped projection of the posterior cruciate bed on a horizontal plane is denoted as A, wherein 10 degrees≦ A ≦ 40 degrees; and/or
an included angle between a projection of the posterior cruciate bed on a vertical plane and a horizontal plane is denoted as B, wherein 20 degrees≤ B≤ 60 degrees.

7. A method for constructing a posterior cruciate bed of a bearing for an artificial knee joint, the posterior cruciate bed of the bearing for the artificial knee joint being the posterior cruciate bed of the bearing for the artificial knee joint according to claim 1, and the method comprising:
defining a first projection plane parallel to a bottom surface of the body, and defining a point, which is adjacent to the outer support portion, of projection points of a junction of the posterior condyle surface and the recess on the first projection plane, as a starting point of a generatrix;
constructing the generatrix which extends obliquely upwards and points to the inner support portion by taking the starting point of the generatrix as an origin; and
generating an entity with a polygonal section according to the constructed generatrix, and cutting the bearing at a junction area between the entity and the bearing by the entity to form the posterior cruciate bed.

8. The method for constructing the posterior cruciate bed of the bearing for the artificial knee joint according to claim 7, wherein the first projection plane is located 2mm to 15mm below a lowest point of an upper surface of the outer support portion; and/or
the polygonal section is round or square.

9. The method for constructing the posterior cruciate bed of the bearing for the artificial knee joint according to claim 7, wherein constructing the generatrix which extends obliquely upwards and points to the inner support portion by taking the starting point of the generatrix as the origin comprises:
defining a plane orthogonal to the first projection plane as a second projection plane, and a center line of the body in the width direction of the body is located at the second projection plane; and
constructing a first projection line forming an included angle a with the second projection plane on the first projection plane, constructing a second projection line forming an included angle b with the first projection plane on the second projection plane, and deducing a spatial position of the generatrix by taking the first projection line and the second projection line as projections of the generatrix.

10. The method for constructing the posterior cruciate bed of the bearing for the artificial knee joint according to claim 9, wherein 5 degrees≦ a ≦ 20 degrees, and 20 degrees≦b≦60 degrees.
